(19)

Europäisches Patentamt

European Patent Office

Office européen des brevets

(11) **EP 4 311 475 A1**

(12) **EUROPEAN PATENT APPLICATION**

(43) Date of publication:
**31.01.2024 Bulletin 2024/05**

(21) Application number: **23163486.6**

(22) Date of filing: **22.03.2023**

(51) International Patent Classification (IPC):
**A61B 3/13** (2006.01)    **A61B 3/117** (2006.01)
**A61B 3/10** (2006.01)

(52) Cooperative Patent Classification (CPC):
**A61B 3/13; A61B 3/1005; A61B 3/1176**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.07.2022 LU 502565**

(71) Applicant: **ELLEX MEDICAL PTY. LTD.
Mawson Lakes, SA 5095 (AU)**

(72) Inventors:
• **Haarhoff, David John
  Highbury, SA 5089 (AU)**
• **Henriksen, Soren
  Clarence Gardens, SA 5039 (AU)**
• **Benson, Eric
  Magill, SA 5072 (AU)**

(74) Representative: **Sattler de Sousa e Brito, Clara
ARROBA Patentanwaltsgesellschaft mbH
Bahnhofstraße 2
65307 Bad Schwalbach (DE)**

(54) **DEVICE AND METHOD FOR DETERMINING LOCATION OF AN OBJECT OF INTEREST WITHIN AN EYE OF A PATIENT, AND OPHTHALMIC APPARATUS**

(57)    The invention relates to a device for determining a location of an object of interest within an eye of a patient, in particular within a vitreous body of the eye, with means for generating at least one optical beam path and with a reference plane opposite the object of interest, the device is set up to determine an object distance between the reference plane and the object of interest at the reference plane.

Fig. 1

## Description

The invention relates to a device for determining a location of an object of interest within an eye of a patient, in particular within a vitreous body of the eye.

The invention further relates to an ophthalmic apparatus for performing a treatment on a patient's eye with an ophthalmic microscope.

The invention also relates to a method for determining a location of an object of interest within an eye of a patient, in particular within a vitreous body of the eye.

From the prior art different ophthalmic apparatuses are known to perform various eye treatments on a patient, for example an ophthalmic microscope for looking into a human eye. Such a microscope consists of many optical components. The microscope has a focal plane located at the focal distance from the objective lens. Objects located at the focal plane will appear in focus to the doctor. The doctor uses the microscope to view anatomical structures within the patient's eye. Certain structures in the eye are easy to locate and focus on, for example, the iris is in the front of the eye and is mostly a planar surface with contrasting colors and structure. The retina (at the back of the eye) is also relatively easy to locate and focus on because it too is mostly planar (when viewed through a microscope), colorful, and structured. Structures in the posterior (behind the iris) of the eye require the doctor so view through the pupil (the aperture in the iris). This is possible with the correct microscope settings and sufficient illumination.

Other structures, such as the cornea, lens, and the vitreous humor or structures within the vitreous humor cannot be easily distinguished because they are transparent or difficult to locate and visualize inside the vitreous humor.

It is the task of this invention to further develop known devices or apparatus and methods for eye examination and/or eye treatment, and in particular to improve a finding of objects within the vitreous humor.

The task of the invention is achieved by a device for determining a position of an object of interest within an eye of a patient, in particular within a vitreous body of the eye, comprising means for generating at least one optical beam path onto the object of interest and a reference plane with respect to the object of interest, wherein the device is set up to determine an object distance between the reference plane and the object of interest at the reference plane.

Since the object distance can be determined at the reference plane, whose position is known, it is also possible to precisely determine the position of the object of interest within the eye.

It is understood that the device in a variety of ways can generate the reference plane so that its location is precisely known. For example, the reference plane is conveniently specified by the means for formulating the reference plane, so that the position of the reference plane is precisely known at the device.

The reference plane can be formulated particularly easily if the reference plane can be formulated using a known reference structure of the patient's eye.

In this respect, the task of this invention is achieved by a device for determining a location of an object of interest within an eye of a patient, in particular within a vitreous body of the eye, relative to a known reference structure especially of the eye, comprising means for generating at least one optical beam path onto the object of interest, the device is set up to determine an object distance between the reference structure and the object of interest at a reference plane formulated by means of the reference structure.

Because the actual object distance between the object of interest and a known reference structure, preferably a known reference structure of the human body, particularly preferably of the human eye, can be determined at a reference plane, the position of the object of interest can be determined particularly easily and reliably.

It is useful to use a known structure of the eye as a reference structure, which can be easily recognized during the eye examination, so that the device can be designed very compactly and can be easily operated by a doctor.

Cumulatively or alternatively, however, it is also possible to define a reference plane independently of a known ocular structure by suitable technical means, opposite and at which the object distance can be determined.

The means for formulating a reference plane can also be provided independently of the present device, for example by an ophthalmic treatment device.

However, the device can be provided to operate more autonomously if the device includes the means for formulating the reference plane.

In the sense of the invention, the term "at the reference plane" means in particular that the object distance between the reference plane and the object of interest is determined along the reference plane. This can take place in the reference plane or also next to the reference plane.

Of relevance to this device, are structures especially within the vitreous, such as vitreous opacities or floaters. So far, these are difficult to locate and visualize. They are normally small, three-dimensional, semitransparent, and "float" in the vitreous. Microscopes have poor depth perception due to their high magnification and consequently short depth of field. As such, it is difficult to find structures that are not planar. Once found, it is difficult to judge where the structure is located relative to other structures of the eye such as the lens or retina. This is particularly important with regards to position along an optical axis. More specifically, once an object which is "floating" in the vitreous is in focus and can be visualized, it is difficult for the doctor to determine how close the object is to either the retina or the posterior of the lens. The short depth of field of the microscope means that the retina will be blurry (out of focus) or not at all distinguishable. The same is true for the lens with the added difficulty being that it is transparent and can't easily be seen.

With the present invention it is easily possible to measure an object distance in this respect, in particular between a focal plane of a microscope resp. the object of interest in the eye and the reference plane.

This measurement can then be reported to the doctor or used for other purposes such as reporting or machine automation purposes.

It was thus recognized that the problem of determining a location of objects of interest within the eye, as explained above, can be achieved by determining the object distance from a defined reference plane, whose position is known, to the object of interest by means of corresponding images at the reference plane.

The object distance between the object of interest and the reference structure or its reference plane can be determined in many ways. For example, by means of value tables or the like, to name just one example of many.

It is particularly advantageous if geometric ray optics are used to describe the object distance.

With regard to a particularly precise and thus preferred construction, it is advantageous if for instance the device is set up to that the object distance to be determinable by triangular geometry of a right-angled triangle which is arranged between the object of interest and the reference structure.

Here, distance sections generated at the reference plane can be put very precisely in relation to the object location at hand.

Thus, for example, a course resp. extension of the distance section can be oriented to the reference plane in the sense of the invention, the reference plane can also be formed only fictitiously.

It is particularly advantageous if the reference plane and a measuring axis or an optical axis of the device enclose a right angle to each other.

For example, the object distance can be determined with the help of angle functions, such as the "Pythagorean theorem" or with other trigonometric functions, if a right-angled triangle is created between the reference structure or especially the reference plane, and the object of interest.

It is understood that the ratios with respect to the object distance, the distance section along the reference plane and the at least one optical path can be related differently.

The hypotenuse, for example, can be generated very easily and more operationally by the at least one beam path, which is directed from the generator source of the generating-means to the object of interest and crosses the reference structure or the reference plane formulated hereof at an angle $\alpha \neq 90°$.

Even at low angles a = 1° to 5° between the at least one optical beam path and the reference plane, the object distance can be determined well with the aid of the optical beam geometry.

However, larger angles a = 10° to 60° or preferred angles a = 30° to 80° are arranged between the at least one optical beam path and the reference plane in order to be able to determine the corresponding object distance more precisely.

Particularly good results can be achieved with preferred angles a = between 74° to 84° or 85° to 89°.

Here, the first cathetus is preferably identical to the object distance, with the fictitious line of the object distance arranged orthogonally to the reference plane.

Preferably, the second cathetus is arranged in such a way that it extends along the reference plane. Advantageously, the second cathetus can be arranged as distance section in the reference plane or alternatively extend as distance section parallel thereto.

In this respect, it is advantageous if the device is set up to that at least one first cathetus of the catheti of a right triangle is formulated by the object distance, that at least one second cathetus of the catheti of the right triangle is formulated at the reference plane, and that the hypotenuse of the right triangle is formulated by the at least one beam path.

As already mentioned, the object distance can be determined in a variety of constructive and procedurally simple ways with the aid of the optical beam geometry.

For example, a distance section provided in the sense of the invention can be defined by two markers generated with respect to the reference plane.

Suitable markers, in turn, can be generated by one or more optical beam paths at the reference plane, advantageously directly by the means for generating the at least one optical beam path of the present device.

In this way, the device comprises, together with the means for generating the at least one beam path, instantly means for generating markers at the reference plane or at a distance section.

In order to detect the markers, it is advantageous if the device has an imaging system for detecting markers at the reference plane.

A suitable optical imaging system can be designed in a particularly simple manner if the imaging system has a camera.

It is understood that an imaging system of any other ophthalmic treatment device may also be used, which has the advantage that the present device may be particularly simple in design.

The optical imaging system conveniently has two, three or more optical beam paths, whereby multiple markers can ideally be generated simultaneously so that the distance section can be determined with less or negligible time delay.

For this purpose, the optical imaging system can advantageously have two or more optical imaging devices.

Optical beam paths can be provided by an optical imaging system, such as a camera or similar.

It is understood that the means for generating the at least one optical beam path, in short also called "generating-means", can be realized differently.

For example, the generating-means can comprise a laser light source whose laser beam is directed at the object of interest and thereby penetrates the reference plane.

Optical beam paths in the sense of the present invention can also be generated and provided by means of light sources, such as laser light sources, or the like.

Accordingly, it is advantageous if the device has suitable technical equipment by means of which corresponding optical beam paths can be generated and provided.

The object distance can thus be determined advantageously in the sense of the invention if the device is set up so that the object distance can be determined by two markers generated at the reference plane by the at least one optical beam path penetrating the reference plane, which markers are arranged separated from each other by a distance section.

The distance section can be determined almost without time delay if markers of two or more optically generated beams are generated or arranged at the reference plane at the same time. This also allows the object distance to be determined in the shortest possible time.

In this design variant, however, two optical beam path sources are required, which are spaced apart along the reference plane.

Here, the device may have two or more generating-means, or alternatively, the device is configured such that a single generating-mean also has two or more such beam path sources.

If applicable, the device also has a deflection device of which an optical beam path of a single beam path source is split into at least two optical partial beam paths, whereby several optical beam paths can be made available simultaneously in a particularly simple manner.

For example, two spaced markers of a single optical path can be used, especially when the reference plane is moved relative to the object of interest.

Cumulatively or alternatively, optical beam paths can be used, which are generated with a time delay and from different angles.

Thus, it is advantageous if markers spaced by a distance section can be generated with a time offset.

An advantage of the latter configurations is that a single optical beam path source is sufficient to determine the object distance in the sense of the invention.

Another advantageous design variant is that the device is set up to that the object distance can be determined by an image shift with respect to markers generated by the at least one optical beam path penetrating the reference plane, which markers spaced apart from each other by a distance section.

Such markers generated by an image shift can have different shapes with respect to the reference plane.

For example, suitable markers can be represented as image information of the object of interest imaged at the reference plane, whereby the distance section between the markers then represents a displacement distance between two image information imaged from different angles and/or two image information imaged at different times.

The markers can also include image information or at least parts thereof.

In any case, the geometric beam optics can be advantageously realized by the present device if the device is set up in such a way that the at least one optical beam path is arranged at an angle a different from 90° with respect to the reference plane, while the object distance is oriented at right angles with respect to the reference plane. Insofar, the distance section between two markers extends along the reference plane.

It is particularly advantageous if the device comprises an optical imaging system for imaging the reference plane and with one or two optical beam paths for imaging images of the object of interest at the reference plane, whereby the device is set up to determine the object distance by an amount of an image shift along the reference plane of images imaged at the reference plane or by a distance section of two optical markers generated at the reference plane. In this way, the present device constructively can easily determine the object distance.

The object distance can be determined particularly accurately if the object distance is proportional to the amount of image shift at the reference plane.

Especially in this context, it is advantageous if images of the object of interest imaged at the reference plane shiftable to further images when the reference plane is shifted relative to the object of interest in order to determine the object distance.

Here, the reference plane is shifted with respect to the object of interest along a fictitious line along the object distance.

It is useful if a distance section between two markers and the object distance correlate with each other in such a way that the object distance can be determined by the distance section. The invention is also characterized by the fact that by the at least one optical beam path suitable markers can be generated at the reference plane, which provide the distance section, which is proportional to the transversely determined object distance, for example to a different focal plane to a microscope, such as a camera focal plane.

The object distance can be determined with particular operational reliability if the device is set up in such a way that a focal plane is arrangeable at the object of interest.

In order to advantageously support the determination of the object distance, the reference plane is preferably displaceable plane-parallel to the focal plane, so that the object distance is arranged to run orthogonally to both planes.

It is advantageous if the device is set up in such a way that optical beam paths in the focal plane can be focused in a common focus.

Alternatively, optical beam paths in the focal plane can be focused at different focal points.

If the device is set up so that the at least one optical beam path to be directed through the reference plane onto the object of interest, markers can be generated more accurately at the reference plane.

Furthermore, it is advantageous if the device is set up to that the at least one optical beam path is arranged at an angle a to the object distance, wherein the object distance is arranged orthogonally to the reference plane. This also contributes to the fact that the object distance can be determined more accurately on the basis of the markers generated with respect to the reference plane or the distance section of the path relating thereto.

The construction of the device can be further improved or simplified if the device is set up to that the object distance comprises an optical axis of the device. The optical axis can be, with respect to a very simple construction, the machine direction of the device, wherein the machine direction is the working direction of the device.

Moreover, the device can be advantageously further developed if the optical axis of the device comprises an optical axis of an observation- and/or treatment device for eyes, such as the optical axis of an ophthalmic microscope or the like.

The object distance can be determined advantageously, in particular with regard to the reference plane, if optical beam paths enclose a viewing angle with each other at least in sections. For example, two optical beam paths that penetrate the reference plane enclose such a viewing angle with each other.

In order for optical markers to be imaged at the reference plane in a way that can be easily determined, it is advantageous if the reference plane is arranged in front of or behind the focal plane with respect to the optical imaging system.

An advantageous embodiment provides that the device has an ophthalmic microscope with a microscope focal length which is in particular identical or at least similar to the focal length of the optical imaging system. This allows the object distance to be determined in a structurally simple and precise manner.

Insofar, the task of the invention is also achieved by an ophthalmic apparatus for performing a treatment on an eye of a patient with an ophthalmic microscope, the ophthalmic apparatus being characterized by a device having at least one of the features described herein. By the inventive device the ophthalmic apparatus is advantageously further developed so that an eye examination or eye treatment can be performed more accurately and more quickly.

The task of the invention is further achieved by a method for operating a device for determining a location of an object of interest within an eye of a patient, in particular within a vitreous body of the eye, wherein at least two optical beam paths cross each other through a reference plane at a common intersection point, wherein the at least two optical beam paths each image the intersection point or a vicinity thereof at the reference plane as spaced images, and wherein the intersection point F and the reference plane are shifted relative to each another, whereby the images are shifted at the reference plane, and the original distance from the intersection point to the reference plane is determined by the amounts of these image shift.

Advantageously, the position of the intersection point as well as the position of a plane passing through this intersection point can be determined relative to the reference plane by measuring a corresponding distance section at the reference plane, and relating this distance section to the object distance to be determined.

Thus, an object distance extending in the machine direction of the device is not measured along its actual longitudinal extension, but by a distance section extending transversely resp. perpendicular thereto. This distance section is measured along its longitudinal sectional extension, which is arranged transverse to this machine direction resp. the object distance.

This means that the object distance searched for can be determined particularly accurately by the distance section at a precisely defined reference plane.

This also applies in particular to the device of which the Method is performed.

It is advantageous if the at least two optical beam paths include a viewing angle with each other, so that the at least two optical beams can cross each other and further generate spaced markers at the reference plane.

This viewing angle is between a value of 0° and 180° so that the optical beam paths can penetrate the reference plane when they subsequently cross at the intersection point.

Preferably, the viewing angle is between a value of 170° and 178°, which enables reliable determination of the object distance at the reference plane.

It is advantageous if the value of the viewing angle is between 140° and 168°. Even with these values, an exact determination of the object distance at the reference plane is possible. Furthermore, the optical beam paths can be arranged well through the pupil of the eye.

It is also useful if the viewing angle is selected in dependence of the position of the intersection point relative to the reference plane.

The task of the invention is further achieved by a method for operating a device for determining a location of an object of interest within an eye of a patient, in particular within a vitreous body of the eye, wherein a reference plane is generated orthogonal to a measuring axis of the device, wherein at least one optical path passes through said reference plane is crossed with said measurement axis at a common intersection point, and wherein the at least one optical path generates a marker at the passage point at the reference plane in order to determine the distance between the intersection point and the reference plane at said reference plane.

The marker can be designed in different ways, as already explained above.

In order to obtain a distance section at the reference plane that can be measured well, it is advantageous if an additional marker is generated at the reference plane.

Preferably, the additional marker is also generated by an optical beam path that penetrates the reference plane.

A process variant provides for optical beam paths are generated successively with a time offset. This allows the process itself to be operated with only a single means for generating at least one optical beam path, which also allows the corresponding device to be constructed with less components.

The distance section at the reference plane between two spaced markers can be easily generated, if the in a time-shifted manner generated optical beam paths are generated in a location-shifted manner as well and include a fictitious viewing angle with each other.

If a focal plane is defined at the intersection point by the two intersecting optical beam paths, the object distance located between two planes can be determined very reliably.

Cumulatively or alternatively, one or more focal planes may also be defined at locations different from the intersection defined by the intersecting optical beam paths, if this appears advantageous.

If the object distance is orthogonal to the two planes, the reference plane and the focal plane are arranged plane-parallel to each other.

The reference plane can be realized very simply at the device in terms of process technology, but also in terms of design, if the reference plane is formulated by at least one camera.

At this point it should be mentioned that alternatively the two optical paths or the at least one optical path and the measurement axis do not necessarily have to intersect with each other, for example if the optical paths or the optical path and the axis are arranged windward to each other.

The task of the invention is also achieved by a method for determining a location of an object of interest within an eye of a patient, in particular within a vitreous body of the eye, wherein an object distance between the object of interest and a known reference structure of the eye is determined at a reference plane formulated by the reference structure.

By means of the known reference structure of the eye, the location of the reference plane is known and the object distance to the object of interest can be accurately determined at the reference plane.

It is advantageous, if the distance, in particular the object distance, is determined by side- and angle ratios of a right-angled triangle.

The task of the invention is also achieved by a special method for determining a location of an object of interest within an eye of a patient, in particular within a vitreous body of the eye, wherein the object of interest is arranged in or near a generated focal plane, wherein a known anatomical eye structure of the eye is arranged in a generated reference plane, wherein at least two optical paths are focused through the reference plane at or in the vicinity of the focal plane, wherein one or more image shifts of at least one image focused with respect to the focal plane are determined at the reference plane, when the focal plane and the reference plane are moved relative to one another, and in which a distance between the focal plane and the reference plane is determined by means of the image shift at the reference plane.

It has been shown that with this method the position resp. location of an object of interest within an eye can be determined simply and yet precisely. For this purpose, a distance section at the reference plane is determined, which results from the measure of the image shift, as described above. The images at the reference plane serve as markers.

By the term "in the vicinity" is meant in the sense of the invention a distance which is less than 10 mm, preferably less than 5 mm or even less, from the actual focal plane.

The task of the invention is also achieved by a further special method for determining a location of an object of interest within an eye of a patient, in particular within a vitreous body of the eye, wherein the object of interest is arranged in or in the vicinity of a generated focal plane, wherein a known anatomical eye structure of the eye is arranged in a generated reference plane, wherein at least one optical beam path is focused through the reference plane at or in the vicinity of the focal plane, and wherein a distance section between two markers generated by the at least one optical beam path at the reference plane is determined, and wherein a distance between the focal plane and the reference plane is determined by the determined distance section at the reference plane.

Also, by this method, the location of the object of interest in the eye can be measured accurately.

The distance or object spacing between the two planes determined in this way can also be referred to as the plane spacing.

On the basis of the now precisely determined object distance, a practitioner resp. doctor can perform an eye treatment.

An advantageous process variant provides that the determined object distance is made available in order to operate a treatment machine automatically. This allows a suitable treatment machine to be operated even more precisely.

In particular, a device or an ophthalmic apparatus or a method for operating a device or a method for determining a location which includes at least one of the following aspects is particularly advantageous:

1. Device (1; 100) for determining a location (2; 102) of an object (3; 103) of interest within an eye (4; 104) of a patient, in particular within a vitreous body (5; 105) of the eye (4; 104), with means (10; 110) for generating at least one optical beam path (11, 12; 111, 112) and with a reference plane (Y) opposite the object (3; 103) of interest, the device (1; 100) is set up to determine an object distance (C) between the reference plane (Y) and the object of interest (3; 103) at the reference plane (Y).

2. Device (1; 100) according to aspect 1, characterized *in that* the reference plane (Y) is formulatable by a known reference structure (23; 123) of the patient eye (4; 104).

3. Device (1; 100) according to aspects 1 or 2, characterized *in that* the device (1; 100) is set up to that the object distance (C) to be determinable by triangular geometry (155) of a right-angled triangle (156) which is arranged between the object (3; 103) of interest and the reference structure (23; 123).

4. Device (1; 100) according to one of aspects 1 to 3, characterized *in that* the device (1; 100) is set up to that at least a first cathetus of the catheti of a right-angled triangle (156) is formulated by the object distance (C), that at least a second cathetus of the catheti of the right-angled triangle (156) is formulated at the reference plane (Y), and that the hypotenuse of the right-angled triangle (156) is formulated by the at least one beam path (11, 12; 111, 112).

5. Device (1; 100) according to any one of aspects 1 to 4, characterized *in that* the device (1; 100) comprises at least one optical imaging system (13, 14; 113, 114) for detecting markers (27, 28; 127, 128) formed at the reference plane (Y).

6. Device (1; 100) according to one of aspects 1 to 5, characterized *in that* the device (1; 100) is set up to that the object distance (C) can be determined by two markers (27, 28; 127, 128) generated at the reference plane (Y) by the at least one optical beam path (11, 12; 111, 112) penetrating the reference plane (Y), which markers (27, 28; 127, 128) are arranged separated from each other by a distance section (B)

7. Device (1; 100) according to aspect 6, characterized *in that* markers (27, 28; 127, 128) spaced apart by a distance section (B) can be generated with a time offset.

8. Device (1; 100) according to one of aspects 1 to 7, characterized *in that* the device (1; 100) is set up to that the object distance (C) can be determined by an image shift with respect to marker (27, 28; 127, 128) generated by the at least one optical beam path (11, 12; 111, 112) penetrating the reference plane (Y), which markers (27, 28; 127, 128) spaced apart from each other by a distance section (B).

9. Device (1; 100) according to one of aspects 1 to 8, characterized by an optical imaging system (13, 14; 113, 114) for imaging the reference plane (Y) and with one or two optical beam paths (11, 12; 111, 112) for imaging images ($O_1$, $O_2$; $O'_1$, $O'_2$) of the object of interest (3; 103) at the reference plane (Y), wherein device (1; 100) is set up to determine the object distance (C) by an amount ($y_1$, $y_2$) of an image shift along the reference plane (Y) of images ($O_1$, $O_2$; $O'_1$, $O'_2$) imaged at the reference plane (Y) or by a distance section (B) of two optical markers (27, 28; 127, 128) generated at the reference plane (Y).

10. Device (1; 100) according to aspect 9, characterized *in that* the object distance (C) is proportional to the amount ($y_1$, $y_2$) of image shift at the reference plane (Y).

11. Device (1; 100) according to one of aspects 1 to 10, characterized *in that* images (O) of the object of interest imaged at the reference plane (Y) are shiftable to further images (O') when the reference plane (Y) is shifted relative to the object (3; 103) of interest in order to determine the object distance (C).

12. Device (1; 100) according to any one of aspects 1 to 11, characterized *in that* a distance section (B) between two markers (27, 28; 127, 128) and the object distance (C) correlate with one another in such a way that the object distance (C) can be determined by means of the distance section (B)

13. Device (1; 100) according to one of aspects 1 to 12, characterized *in that* the device (1; 100) is set up to that a focal plane (E) is arrangeable at the object of interest (3; 103)

14. Device (1; 100) according to one of aspects 1 to 13, characterized *in that* optical beam paths (11, 12; 111, 112) are focusable in the focal plane (E) in a common focus (F).

15. Device (1; 100) according to one of aspects 1 to 13, characterized *in that* optical beam paths (11, 12; 111, 112) are focusable in the focal plane (E) at different focal points.

16. Device (1; 100) according to one of aspects 1 to 15, characterized *in that* the device (1; 100) is set up to that the at least one optical beam path (11, 12; 111, 112) to be directed through the reference plane (Y) onto the object of interest (3; 103).

17. Device (1; 100) according to one of aspects 1 to 16, characterized *in that* the device (1; 100) is set up to that the at least one optical beam path (11, 12; 111, 112) is arranged at an angle (α) to the object distance (C), wherein the object distance (C) is arranged orthogonally to the reference plane (Y)

18. Device (1; 100) according to one of aspects 1 to 17, characterized *in that* the device (1; 100) is set up to that the object distance (C) comprises an optical axis (8; 108) of the device (1; 100).

19. Device (1; 100) according to one of aspects 1 to 18, characterized *in that* optical beam paths (11, 12; 111, 112) enclose a viewing angle (θ) with each other at least in sections.

20. Device (1; 100) according to one of aspects 1 to 19, characterized *in that* the reference plane (Y) is arranged in front of or behind the focal plane (E) with respect to the optical imaging device (13, 14; 113, 114).

21. Device (1; 100) according to one of aspects 1 to 20, characterized *in that* the device (1; 100) comprises an ophthalmic microscope (150) having a microscope focal length (f) which is in particular identical to the focal length (f) of the optical imaging device (13, 14; 113, 114).

22. Ophthalmic apparatus (150A) for performing treatment on an eye (4; 104) of a patient with an ophthalmic microscope (150), characterized by an apparatus (1; 100) according to one of the preceding aspects.

23. Method for operating a device (1; 100) for determining a location (2; 102) of an object (3; 103) of interest within an eye (4; 104) of a patient, in particular within a vitreous body (5; 105) of the eye (4; 104), in particular for operating the device (1; 100) according to one of aspects 1 to 21, wherein at least two optical paths (11, 12; 111, 112) cross each other through a reference plane (Y) at a common intersection point (F), wherein the at least two optical paths (11, 12; 111, 112) each image the intersection point (F) or a vicinity thereof at the reference plane (Y) as spaced images ($O_1$, $O_2$; $O'_1$, $O'_2$), and wherein the intersection point (F) and the reference plane (Y) are shifted relative to each other, whereby the images ($O_1$, $O_2$; $O'_1$, $O'_2$) are shifted relative to one other at the reference plane (Y), and the original distance (C) from the intersection point (F) to the reference plane (Y) is determined by the amounts of these image shift.

24. Method according to aspect 23, characterized *in that* the at least two optical beam paths (11, 12; 111, 112) include a viewing angle (θ) with each other.

25. Method for operating a device (1; 100) for determining a location (2; 102) of an object of interest (3; 103) within an eye (4; 104) of a patient, in particular within a vitreous body (5; 105) of the eye (4; 104), in particular for operating the device (1; 100) according to one of aspects 1 to 21, wherein a reference plane (Y) orthogonal to a measuring axis (6; 106) of the device (1; 100) is generated; 100), wherein at least one optical beam path (11, 12; 111, 112) passes through this reference plane (Y) is crossed with the measurement axis (6; 106) at a common intersection point (F), and wherein the at least one optical beam path (11, 12; 111, 112) generates a marker (27, 28; 127, 128) at the passage point at the reference plane (Y) in order to determine the distance (C) between the intersection point (F) and the reference plane (Y) at said reference plane (Y).

26. Method according to aspect 25, characterized *in that* an additional marker (27, 28; 127, 128) is generated at the reference plane (Y).

27. Method according to one of aspects 23 to 26, characterized *in that* optical beam paths (11, 12; 111, 112) are generated successively with a time offset.

28. Method according to aspect 27, characterized *in that* the in a time-shifted manner generated optical beam paths (11, 12; 111, 112) are generated in a location-shifted manner as well and include a fictitious viewing angle with each other.

29. Method according to aspects 23 to 28, characterized *in that* a focal plane (E) is defined at the intersection point (F) by the two intersecting optical beam paths (11, 12; 111, 112).

30. Method according to aspects 23 to 29, characterized *in that* the reference plane (Y) is formulated by at least one camera unit (15, 16; 115, 116).

31. Method for determining a location (2; 102) of an object (3; 103) of interest within an eye (4; 104) of a patient, in particular within a vitreous body (5; 105) of the eye (4; 104), wherein an object distance (C) between the object (3; 103) of interest and a known reference structure (23; 123) of the eye (4; 104) is determined at a reference plane (Y) formulated by the reference structure (23; 123).

32. Method according to one of aspects 23 to 31, characterized *in that* the distance, in particular the object distance (C), is determined by side- and angle ratios of a right-angled triangle (156).

33. Method for determining a location (2; 102) of an object (3; 103) of interest within an eye (4; 104) of a patient, in particular within a vitreous body (5; 105) of the eye (4; 104), wherein the object (3; 103) of interest is arranged in or near a generated focal plane (E), wherein a known anatomical eye structure (22; 122) of the eye (4; 104) is arranged in a generated reference plane (Y), wherein at least two optical paths (11, 12; 111, 112) are focused through the reference plane (Y) at or in the vicinity of the focal plane (E), wherein one or more image shifts of at least one image (O) focused with respect to the focal plane (E) are determined at the reference plane (Y), when the focal plane (E) and the reference plane (Y) are moved relative to one another, and in which a distance (C) between the focal plane (E) and the reference plane (Y) is determined by means of the image shift at the reference plane (Y).

34. Method for determining a location (2; 102) of an object (3; 103) of interest within an eye (4; 104) of a patient, in particular within a vitreous body (5; 105) of the eye (4; 104), wherein the object (3; 103) of interest is arranged in or near a generated focal plane (E), wherein a known anatomical eye structure (22; 122) of the eye (4; 104) is arranged in a generated reference plane (Y), wherein a known anatomical eye structure (22; 122) of the eye (4; 104) is arranged in a generated reference plane (Y), wherein at least one optical beam path (11, 12; 111, 112) is focused through the reference plane (Y) at or in the vicinity of the focal plane (E), and wherein a distance section (B) between two markers generated by the at least one optical beam path (11, 12; 111, 112) at the reference plane (Y) is determined, and wherein a distance (C) between the focal plane (E) and the reference plane (Y) is determined by the determined distance section (B) at the reference plane (Y).

35. Method according to one of aspects 23 to 34, characterized *in that* the determined object distance (C) is provided to operate a treatment machine in an automated manner.

It should also be noted that in the context of the present patent application indefinite articles and indefinite numerical indications such as "one...", "two..." etc. are generally to be understood as at least indications, i.e. as "at least one...", "at least two..." etc., unless it is clear from the context or the specific text of a particular passage that only "exactly one...", "exactly two..." etc. is meant.

At this point it should also be mentioned that in the context of the present patent application the term "in particular" is always to be understood in such a way that with this expression an optional, preferred feature is introduced. The term is not to be understood as "in fact" and not as "namely".

At this point it is also claimed that the described methods can be supplemented by further technical features described here, in particular by features of the device, in order to advantageously further develop the methods or to be able to represent or formulate method specifications even more precisely.

It is understood that the features of the solutions described above or in the claims can also be combined, if necessary, in order to be able to implement the advantages and effects achievable herein in a correspondingly cumulative manner.

In addition, further features, effects and advantages of the present invention are explained with reference to the accompanying drawing and the following description, in which exemplary first devices for determining a position of an object of interest are illustrated and described.

Components which at least essentially correspond in terms of their function in the individual figures can be identified here with the same reference signs, whereby the components do not have to be numbered and explained in all figures.

The following figures show:

Figure 1    schematically a model view of a first device for determining a location of an object of interest with a dual imaging system;

Figure 2    schematically another view of the first device of figure 1;

Figure 3    schematically another model view of an alternative device for determining a location of an object of interest with integrated ophthalmic microscope;

Figure 4    schematically a detailed view of the alternative device shown in figure 3 with the reference plane through the known reference structure of the iris;

Figure 5    schematically another model view of another device for determining a location of an object of interest with means comprising a twin camera;

Figure 6    schematically another model view of another device for determining a location of an object of interest with a device comprising a depth camera for time-of-flight measurement;

Figure 7    schematically another model view of another device for determining a location of an object of interest with means comprising slit lamp translation tracking;

Figure 8    schematically another model view of another device for determining a position of an object of interest with means comprising at least one camera for depth perception;

Figure 9 schematically another model view of another device for determining a location of an object of interest comprising means for printing a pattern on a contact lens and means comprising a camera for focusing said pattern; and

Figure 10 schematically another model view of another device for determining a location of an object of interest with a device comprising an OCT-detection of the object of interest or an Ultrasound A-scan.

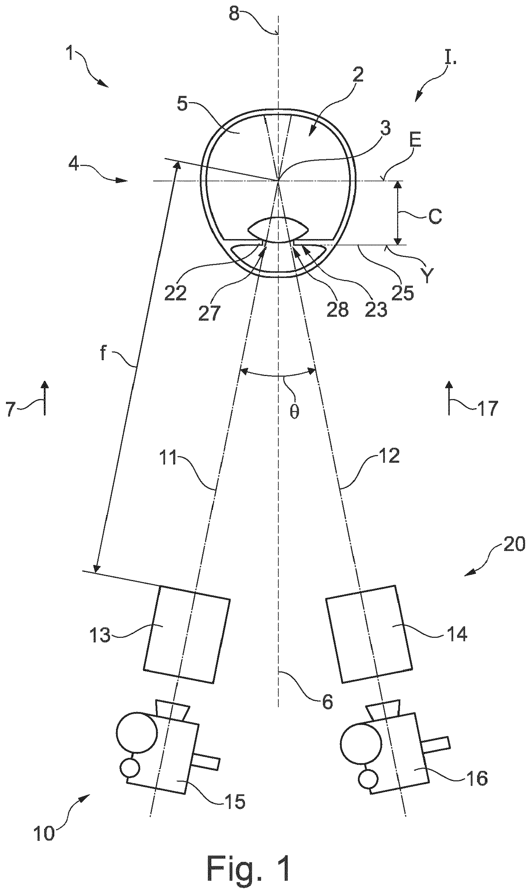

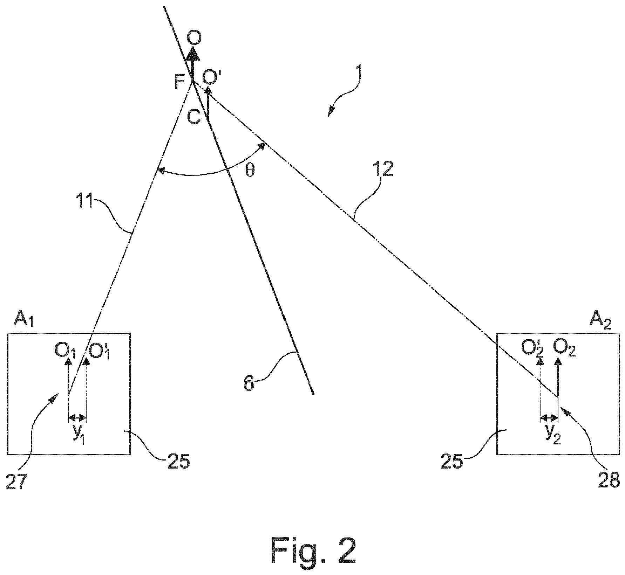

The device 1 shown in figures 1 and 2 is a first embodiment example I. of the invention for determining a position 2 of an object 3 of interest within an eye 4 of a patient not shown in detail here.

The object 3 of interest is a foreign body structure, such as a so-called floater or the like, within the eye 4, more precisely within the vitreous body 5 of the eye 4.

The device 1 has a machine axis 6 in machine direction 7, in the longitudinal extension of which an optical axis 8 of the device 1 can be defined.

Moreover, the device 1 has means 10 for generating two optical beam paths 11 and 12.

In this case, the means 10 of generating comprises two imaging systems 13 and 14 with two camera units 15 and 16.

The optical axis 8 of the device 1 is defined here by the viewing direction 17 of the imaging systems 13, 14. The viewing direction 17 is in machine direction 7.

Further, the device 1 has means 20 for formulating a reference plane Y, which can be realized by the imaging systems 13, 14 or the camera units 15, 16.

In this first embodiment, the reference plane Y is laid as a sectional plane (not numbered again) through a known eye structure 22, the known eye structure 22 being here the iris (not numbered again) of the eye 4 and thus representing a known reference structure 23.

The two optical paths 11 and 12 enclose a viewing angle $\theta$ with each other, so that the two optical paths 11 and 12, coming from different directions, cross at a common intersection point F.

The distance between the imaging systems 13, 14 and the intersection point F define the focal length f of the device 1.

In this first embodiment, the intersection point F defines the focal point F of a focal plane E.

In this first embodiment, the intersection point F is selected in such a way that the object 3 of interest is also located there.

Furthermore, the two optical beam paths 11, 12 and the reference plane Y are arranged with respect to each other in such a way that the two optical beam paths 11 and 12 pass through the reference plane Y.

In order to determine the position 2 of the object 3 of interest within the eye 4, the object distance C between the intersection point F and the reference structure 23 resp. between the focal plane E and the reference plane Y is now determined.

Especially figure 1 shows a simplified schematic of a device 1 and a cross-section of the eye 4. The two imaging systems 13, 14 of device 1 are arranged to focus on the same location, intersection F, but are separated by the viewing angle $\theta$.

In this embodiment example, the two imaging systems 13, 14 are the same and comprise suitable optical components to image the focal plane E at the intersection F.

The camera unit 15 resp. 16 has been added to each imaging system 13, 14 to capture the images formed. Objects located near the focal plane E will be imaged by the two imaging systems 13, 14 to produce two similar images $O_1$ and $O_2$ (see also figure 2), the images $O_1$ and $O_2$ will share the same content and have similar sizes and spatial arrangement.

However, for objects located behind or in front of the focal plane E, for example the iris (object of interest 3) located an object distance C in front of the focal point F, the images $O_1$ and $O_2$ will differ.

The two images $O_1$ and $O_2$ will still share the same content, have similar sizes and spatial arrangement, but the content will be shifted by an amount proportional to the object distance C, as shown in more detail in figure 2.

Figure 2 shows another schematic of the device 1, whereby the imaging system 13, 14 and camera units 15, 16 are removed for simplicity, and the schematic is a 3D view.

The two squares $A_1$ and $A_2$ represent the image plane 25 at the reference plane Y of the two imaging systems 13 and 14.

The imaging systems 13 and 14 produce images of an object (large solid vertical arrow labeled O) located at their common focal point F. The two images of O are labeled $O_1$ and $O_2$.

When the object 3 of interest is moved an object distance C toward the two imaging systems 13 and 14, as indicated by the large dashed arrow labeled O', the images $O_1$ and $O_2$ generated by O' shift on the image planes 25 to form new images located at $O'_1$ and $O'_2$.

The image shift is labeled $y_1$ and $y_2$.

Since the viewing angle $\theta$ is fixed, the amount of displacement $y_1$ and $y_2$ can be directly correlated with the object distance C.

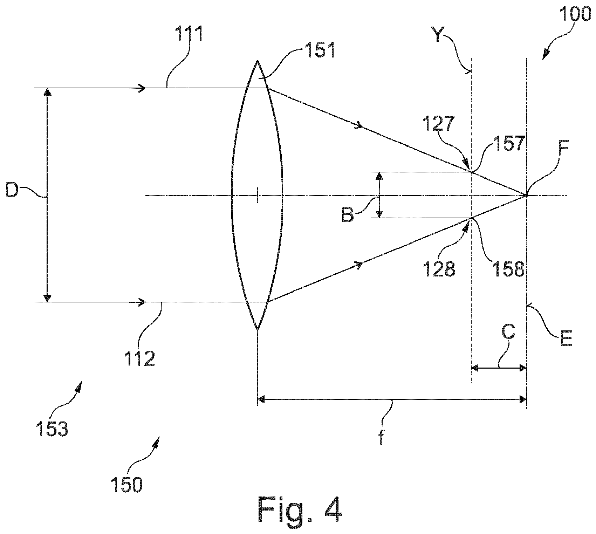

The images $O_1$ and $O_2$ as well as the shifted images $O'_1$ and $O'_2$ can be considered markers 27, 28 at the reference plane Y or include appropriate markers 27, 28 at the reference plane Y, whereby the markers 27 and 28 are spaced apart from each other by a distance space B (not shown here, cf. figure 4).

The object distance C can thus be easily and very exact determined at the reference plane Y.

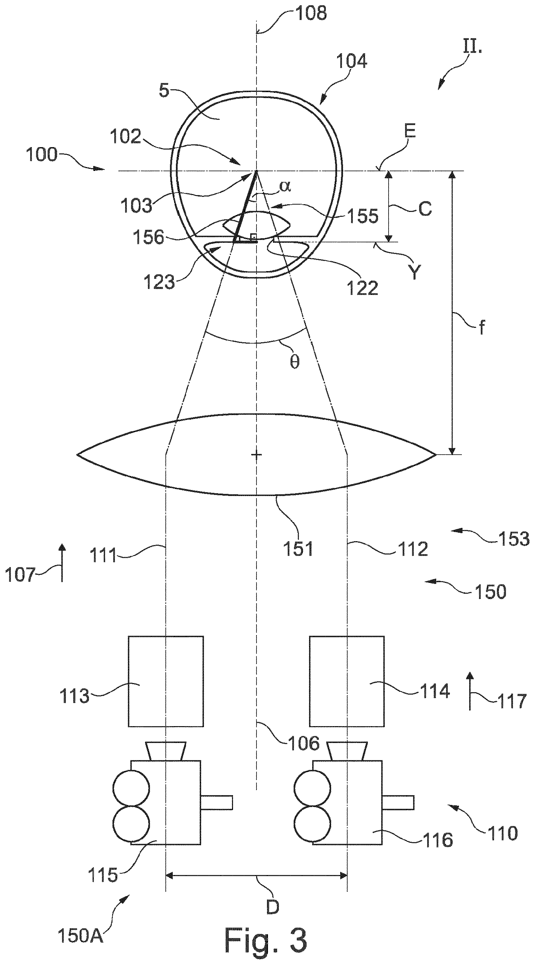

Preferably, the object distance C between the front of the iris and the focal plane E of an ophthalmic microscope is 150 (see figure 3) determined, but other reference structures 23 of a patient's eye 4 can also be used.

Insofar, figure 3 shows another embodiment example II. of an alternative device 100 for determining a location 102 of an object 103 of interest within an eye 104 of a patient, in particular within a vitreous body 105 of the eye 104, which are similar to the device 1 shown in the Figures 1 and 2, but the device 100 has an ophthalmic microscope 150 in addition. More specifically, the alternative device 100 in this further embodiment example has been integrated into the ophthalmic microscope 150.

The ophthalmic microscope 150 is illustrated in figure 3 only by its objective lens 151.

The alternative device 100 has a machine direction 107 as well, in the longitudinal extension of which machine axis 106 resp. an optical axis 108 of the alternative device 100 can be defined.

Figure 3 shows another schematic of the alternative device 100 and a cross-section of the patient's eye 104 with an iris used as a reference structure 123 resp. as a known eye structure 122. Figure 3 is oriented with the patient (not shown, only the patient's eye 104) at the top of the Figure 3.

In this configuration, two imaging systems 113 and 114 share the microscope's objective lens 151 and are configured such that their focal planes (not separately numbered) coincide with that of the ophthalmic microscope's 150, namely in one common focal plane E.

In this further embodiment example, the imaging systems 113 and 114 are located parallel to each other, whereby a binocular viewing path 153 with two parallel optical beam paths 111 and 112 of the ophthalmic microscope's 150, which are separated by a beam path distance D.

The optical beam paths 111, 112 (generated by means 110 for generating optical beam paths) are arranged parallel to the optical axis 108 of the device 100 up to the objective lens 151, and in this embodiment, the optical axis 108 of the device 100 is formed by the ophthalmic microscope 150. The refraction power of objective lens 151 creates the viewing angle θ between the two optical beam paths 111 and 112 after the objective lens 151.

The exact configuration of the alternative device 100 resp. of the imaging systems 113, 114 and the cameras units 115, 116 can vary, they could be configured to sample light along the binocular viewing path 153 using other beam splitters (not shown), or they could be located out of the viewing paths 153, their individual optical beam paths 111, 112 could be folded by mirrors (not shown) or other optical components (not shown) to make them fit within the ophthalmic microscope 150.

The key is that they both image the same object near the focal plane E from different angles.

Anyway, the camera units 115, 116 will digitize the two images $O_1$ and $O_2$ (cf. figure 2). These digital images $O_1$ and $O_2$ are then processed via a processor running software algorithms and code.

The images $O_1$ and $O_2$ are compared and the difference in position of the same object in each image $O_1$ and $O_2$ is found. It is this difference that is used to determine the object distance C that object is from the reference plane Y.

The devices 1 and 100 described above uses two camera units 15, 16 resp. 115, 116, however it is conceivable for a device 1 or 100 to only use one camera unit 15 or 16 resp. 115 or 116, to collect both images $O_1$ and $O_2$.

The key is that the two images $O_1$ and $O_2$ are of the same object but imaged from different angles, and at least one of the images $O_1$ and $O_2$ needs to be at a different angle from the machine axis 6 resp. microscope's optical axis 8 resp. 108, the axis 6, 8 or 108 along which especially the ophthalmic microscope 150 is moveable.

In generally, it is not necessary that the image systems 13, 14 resp. 113, 114 and ophthalmic microscope 150 share the same focal plane E.

The imaging systems 13, 14 resp. 113, 114 can have a different focal plane E located at a position better suited for imaging the structure of the eye 4 resp. 104 of interest, for example the iris.

The relative distance C between the planes E, Y can be accounted for in the software.

Furthermore, a triangular geometry 155 of a right-angled triangle 156 is arranged between the object of interest 103 and the reference structure 123, by means of which the object distance C can be determined with the aid of angular functions. Here, the optical path 111 is positioned behind the objective lens 151 at an angle a with respect to the machine axis 106 or optical axis 108.

The device 100 is integrated together with the ophthalmic microscope 150 in an ophthalmic apparatus 150A.

Further alternative concepts are explained on the basis of the following description.

Figure 4 shows the same device 100, which is shown in Figure 3, with the ophthalmic microscope 150, but instead of the eye 104, an intersecting plane resp. reference plane Y has been placed at the location of the iris, whereby the iris is a known eye structure 122 (cf. figure 3).

Additionally, the two optical beam path 111 and 112 are projected from inside the ophthalmic microscope 150 and onto the focal plane E.

The two optical beam paths 111 and 112 represent two narrow beams of light, which are located a beam path distance D apart at the objective lens 151 and are positioned to cross at the focal plane E.

The two optical beam paths 111 and 112 of light will project two spots 157 and 158 at the reference plane Y, which are separated by a distance section B.

These spots 157 and 158 can be considered as markers 127, 128, with the markers 127 and 128 spaced apart from each other by a distance section B at the reference plane Y.

By way of geometry, B is related to C by the following mathematical relationship:

$$B = D/F \times C \quad => \qquad C = F/D \times B$$

Insofar the required object distance C can be easily determined by the alternative device 100, too.

Especially an imaging system 113, 114, comprising a camera unit 115, 116, optical components, like the objective lens 151 or the like, and a computer, can measure the distance section B between the two spots 157 and 158 resp. the markers 127 128 on the reference plane Y and thereby determine the object distance C.

Some further notes regarding the design implementation of devices 1, 100 of the invention.

The arrangements of the embodiment examples described above made use of two rays of light resp. of two optical beam path 111, 112 thereof, and their relative separation (spots 157, 158 resp. markers 127, 128) at the reference plane Y to determine the object distance C of the reference plane Y in front of the focal plane E. Several alternatives configuration to this can be used.

Alternative 1:

More than two rays of light resp. optical beams paths 111, 112 could be used.

For example, three or more optical beam paths 111, 112 can be used to make more spots than two spots 157, 158 resp. markers than two markers 127, 128 at the reference plane Y. This arrangement may require more measurements and an average value to be used. Such an arrangement may have advantages, for example, it could counter any small angles or curvature of the reference plane Y resp. intersecting plane.

Alternative 2:

Instead of two rays resp. optical beam paths 111, 112, a single beam of size A (diameter of circular cross-section) could be used, the single beam could be focused to a small spot at the focal plane E. In this arrangement, the measurement B would not be the separation but rather the beam size A (diameter of circular cross-section) on the reference plane Y resp. intersecting plane.

Alternative 3:

Instead of beams of light (optical beam path 11, 12; 111, 112) with circular cross section, other shaped beams could be used. For example, beams with a triangular, or arrowhead cross-sectional shape. A further note, beams, whose cross-sectional shape is not of symmetry 1 (e.g., a triangle) may have an advantage, as the projected image on the reference plane Y will invert if the reference plane Y is behind or in front of the focal plane E.

Alternative 4:

Instead of beams of light (optical beam path 11, 12; 111, 112) with circular cross section, patterned shapes (such as parallel lines, concentric circles, or other suitable pattern) could be projected onto the reference plane Y. The patterns could be slightly angled from each other to cause visual fringes (Moire Patterns) as they overlap. The fringes can be used to make very precise measurements of distance.

Alternative 5:

Instead of the two beams 11, 12; 111, 112 converging to a single point, like the intersection point resp. focal point F, at the focal plane E, they could be configured to have separated points at the focal plane E. This can be achieved by directing them differently from within the ophthalmic microscope 150. Such a configuration may be advantageous if there exists an optimal separation distance section B for the imaging system 13, 14; 113, 114 to measure B. If so, the device 1, 100 could be configured to ensure the distance section B fell within the optimal range for appropriate values of the object distance C.

Alternative 6:

The beams (optical beam path 11, 12; 111, 112) could be focused to a different plane to that of the microscopes focal plane E. For example, this plane could be located at the average position of the reference plane Y (when viewing into the vitreous). This may have the advantage that the images ($O_1$ and $O_2$ as well as $O'_1$ and $O'_2$) produced by the beams (optical beam path 11, 12; 111, 112) at the reference plane Y would be in sharp focus, this would potentially improve the imaging systems 13, 14; 113, 114 ability to measure the distance section B.

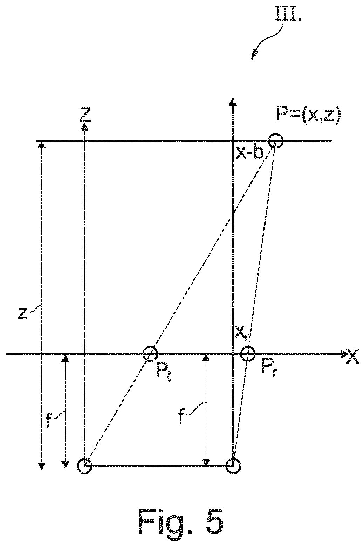

Other alternative embodiment examples of how the devices 1, 100 explained here can be alternatively designed or supplemented in terms of construction are shown schematically with reference to the following Figures 5 to 10, although these corresponding alternative concepts of the devices are not shown in further detail: A first other alternative embodiment example III. is shown by figure 5, and this first other alternative embodiment example based on a horizontal stereo methodology. The basic idea is that a twin camera takes pictures of the same scene, in two different positions, and recovers the depth at each pixel. One advantage here is the ability to determine a safe treatment area by measuring the disparity of the images taken by the cameras. The system can achieve a good resolution and repeatability by measuring the laser depth perception.

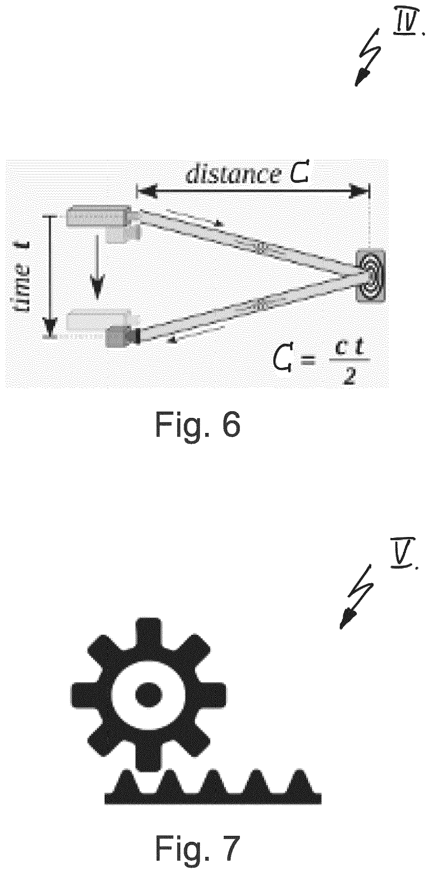

A second other alternative embodiment example IV. is shown by figure 6, and this second other alternative embodiment example based on using a depth camera that uses time of flight measurement. The object distance C is determined on the basis of the measured time of flight of a light beam between a device for generating at least one suitable optical beam path and a detector unit spaced therefrom, such as a camera unit or the like, where the object distance $C = \frac{1}{2} \times c \times t$, where c = speed of light and t = time of flight. A suitable computer program is used to take into account the time delay of a light pulse from a laser or LED that is reflected from a reflective curved surface such as the cornea.

A third other alternative embodiment example V. is shown by figure 7, and this third other alternative embodiment example based on slit-lamp translation tracking, which can track the movement of a laser delivery head in each direction, sensed by a mechanical to the electrical sensor. Sensor signals are captured by the CPU and processing by software to trace location. One advantage is that this construction is easy to implement. It can be cost-effective if there are no step motors modules involved.

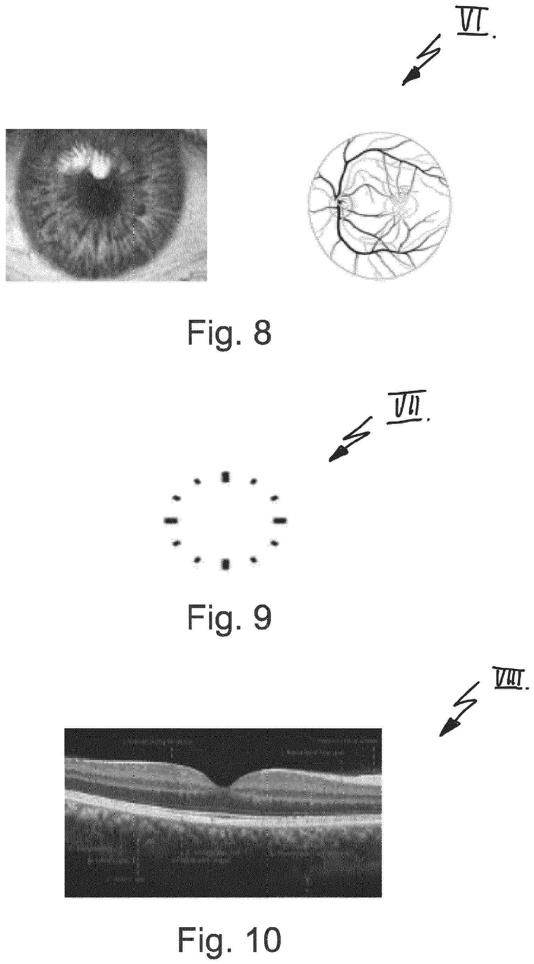

A fourth other alternative embodiment example VI. is shown by figure 8, and this fourth other alternative embodiment example based on depth perception in a single camera. No focus adjustment is done. If the retina image is in focus display a warning or sets a measurement reference. Another or the same camera can be used to detect proximity to the iris and give a warning if the iris is almost in focus. Here, in particular, there is the ability to determine a safe treatment area by identifying the iris/crystalline lens and retina as points of reference.

Another method could be to defocus blur analysis to estimate depth perception software detects the image blurriness and at the same time commands the liquids lens or moveable lens to correct the image. The current applied to the liquid lens or moveable lens to do the image correction is read by the software to quantify how much depth variation the target "Eye" has displaced. One advantage here is to defocus blur analysis can provide a strong relationship to detecting depth perception.

A fifth other alternative embodiment example VII. is shown by figure 9, and this fifth other alternative embodiment example based on printing a pattern on contact lens or its surrounding and focusing a camera on them. An advantage here is: Having a marking reference to the cornea can be a strong reference detected by the camera to perceive depth.

A sixth other alternative embodiment example VIII. is shown by figure 10, and this sixth other alternative embodiment example based on using OCT (Optical Coherence Tomography) to detect floater positions or Ultrasound-A-Scan. One advantage herby is that OCT can provide the accuracy and reliability for this purpose.

All of the above-described alternative embodiments or concepts solve the present problem of the invention even without the other features of the invention.

However, the alternative embodiment examples or concepts described can also be combined with the other features in order to further develop devices in the sense of the invention.

At this point, it should be explicitly noted that the features of the solutions described above or in the claims and/or figures can also be combined, if necessary, in order to be able to implement or achieve the explained features, effects and advantages in a correspondingly cumulative manner.

It is understood that the embodiment examples explained above are only first embodiments of the device according to the invention. In this respect, the embodiment of the invention is not limited to these embodiments.

All of the features disclosed in the application documents are claimed to be essential to the invention, provided that they are new, individually or in combination, compared to the prior art.

**List of reference signs:**

| | |
|---|---|
| 1 | device |
| 2 | location resp. position |
| 3 | object of interest |
| 4 | eye |
| 5 | vitreous body |
| 6 | machine axis resp. measuring axis |
| 7 | machine direction |
| 8 | optical axis |
| 10 | means for generating at least one optical beam path |
| 11 | first optical beam path |
| 12 | second optical beam path |
| 13 | first imaging system |
| 14 | second imaging system |
| 15 | first camera unit |
| 16 | second camera unit |
| 17 | viewing direction |
| 20 | means for formulating a reference plane |
| 22 | known eye structure |
| 23 | known reference structure |
| 25 | image plane |
| 27 | first marker |
| 28 | spaced further marker |
| | |
| 100 | alternative device |
| 102 | location resp. position |
| 103 | object of interest |
| 104 | eye |
| 105 | vitreous body |
| 106 | machine axis resp. measuring axis |
| 107 | machine direction |
| 108 | optical axis |
| 110 | means for generating at least one optical beam path |

| 111 | first optical beam path |
| 112 | second optical beam path |
| 113 | first imaging system |
| 114 | second imaging system |
| 115 | first camera unit |
| 116 | second camera unit |
| 117 | viewing direction |
| 122 | known eye structure |
| 123 | known reference structure |
| 127 | first marker |
| 128 | spaced further marker |
| 150 | ophthalmic microscope |
| 150A | ophthalmic apparatus |
| 151 | objective lens |
| 153 | binocular viewing path |
| 155 | triangular geometry |
| 156 | - right-angled triangle |
| 157 | first spot |
| 158 | further spot |

| f | focal length |
| B | distance section |
| C | object distance |
| D | beam path distance |
| E | focal plane |
| F | intersection point |
| Y | reference plane resp. intersection plane |
| $\theta$ | viewing angle |
| a | angle |

| I. | embodiment example |
| II. | second embodiment example |
| III. | first alternative embodiment example |
| IV. | second alternative embodiment example |
| V. | third alternative embodiment example |
| VI. | fourth alternative embodiment example |
| VII. | fifth alternative embodiment example |
| VIII. | sixth alternative embodiment example |

**Claims**

1. Device (1; 100) for determining a location (2; 102) of an object (3; 103) of interest within an eye (4; 104) of a patient, in particular within a vitreous body (5; 105) of the eye (4; 104), with means (10; 110) for generating at least one optical beam path (11, 12; 111, 112) and with a reference plane (Y) opposite the object (3; 103) of interest, the device (1; 100) is set up to determine an object distance (C) between the reference plane (Y) and the object of interest (3; 103) at the reference plane (Y).

2. Device (1; 100) according to claim 1, *characterized in that* the device (1; 100) is set up to that the object distance (C) to be determinable by triangular geometry (155) of a right-angled triangle (156) which is arranged between the object (3; 103) of interest and the reference structure (23; 123) , whereby in particular the device (1; 100) is set up to that at least a first cathetus of the catheti of a right-angled triangle (156) is formulated by the object distance (C), that at least a second cathetus of the catheti of the right-angled triangle (156) is formulated at the reference plane (Y), and that the hypotenuse of the right-angled triangle (156) is formulated by the at least one beam path (11, 12; 111, 112).

3. Device (1; 100) according to claim 1 or 2, *characterized in that* the device (1; 100) comprises at least one optical imaging system (13, 14; 113, 114) for detecting markers (27, 28; 127, 128) formed at the reference plane (Y).

4. Device (1; 100) according to one of claims 1 to 3, *characterized in that* the device (1; 100) is set up to that the object distance (C) can be determined by two markers (27, 28; 127, 128) generated at the reference plane (Y) by the at least one optical beam path (11, 12; 111, 112) penetrating the reference plane (Y), which markers (27, 28; 127, 128) are arranged separated from each other by a distance section (B), whereby in particular markers (27, 28; 127, 128) spaced apart by a distance section (B) can be generated with a time offset.

5. Device (1; 100) according to one of claims 1 to 4, *characterized in that* the device (1; 100) is set up to that the object distance (C) can be determined by an image shift with respect to marker (27, 28; 127, 128) generated by the at least one optical beam path (11, 12; 111, 112) penetrating the reference plane (Y), which markers (27, 28; 127, 128) spaced apart from each other by a distance section (B).

6. Device (1; 100) according to one of claims 1 to 5, *characterized by* an optical imaging system (13, 14; 113, 114) for imaging the reference plane (Y) and with one or two optical beam paths (11, 12; 111, 112) for imaging images ($O_1$, $O_2$; $O'_1$, $O'_2$) of the object of interest (3; 103) at the reference plane (Y), wherein device (1; 100) is set up to determine the object distance (C) by an amount ($y_1$, $y_2$) of an image shift along the reference plane (Y) of images ($O_1$, $O_2$; $O'_1$, $O'_2$) imaged at the reference plane (Y) or by a distance section (B) of two optical markers (27, 28; 127, 128) generated at the reference plane (Y) , whereby in particular the object distance (C) is proportional to the amount ($y_1$, $y_2$) of image shift at the reference plane (Y).

7. Device (1; 100) according to one of claims 1 to 6, *characterized in that* images (O) of the object of interest imaged at the reference plane (Y) are shiftable to further images (O') when the reference plane (Y) is shifted relative to the object (3; 103) of interest in order to determine the object distance (C) , whereby in particular a distance section (B) between two markers (27, 28; 127, 128) and the object distance (C) correlate with one another in such a way that the object distance (C) can be determined by means of the distance section (B.

8. Device (1; 100) according to one of claims 1 to 7, *characterized in that* the device (1; 100) is set up to that the at least one optical beam path (11, 12; 111, 112) to be directed through the reference plane (Y) onto the object of interest (3; 103) , whereby in particular the device (1; 100) is set up to that the at least one optical beam path (11, 12; 111, 112) is arranged at an angle ($\alpha$) to the object distance (C), wherein the object distance (C) is arranged orthogonally to the reference plane (Y).

9. Device (1; 100) according to one of claims 1 to 8, *characterized in that* the device (1; 100) is set up to that the object distance (C) comprises an optical axis (8; 108) of the device (1; 100).

10. Device (1; 100) according to one of claims 1 to 9, *characterized in that* optical beam paths (11, 12; 111, 112) enclose a viewing angle ($\alpha$) with each other at least in sections.

11. Ophthalmic apparatus (150A) for performing treatment on an eye (4; 104) of a patient with an ophthalmic microscope (150), *characterized by* an apparatus (1; 100) according to one of the preceding claims.

12. Method for operating a device (1; 100) for determining a location (2; 102) of an object (3; 103) of interest within an eye (4; 104) of a patient, in particular within a vitreous body (5; 105) of the eye (4; 104), in particular for operating the device (1; 100) according to one of claims 1 to 10, wherein at least two optical paths (11, 12; 111, 112) cross each other through a reference plane (Y) at a common intersection point (F), wherein the at least two optical paths (11, 12; 111, 112) each image the intersection point (F) or a vicinity thereof at the reference plane (Y) as spaced images ($O_1$, $O_2$; $O'_1$, $O'_2$), and wherein the intersection point (F) and the reference plane (Y) are shifted relative to each other, whereby the images ($O_1$, $O_2$; $O'_1$, $O'_2$) are shifted relative to one other at the reference plane (Y), and the original distance (C) from the intersection point (F) to the reference plane (Y) is determined by the amounts of these image shift.

13. Method for operating a device (1; 100) for determining a location (2; 102) of an object of interest (3; 103) within an eye (4; 104) of a patient, in particular within a vitreous body (5; 105) of the eye (4; 104), in particular for operating the device (1; 100) according to one of claims 1 to 10, wherein a reference plane (Y) orthogonal to a measuring axis (6; 106) of the device (1; 100) is generated; 100), wherein at least one optical beam path (11, 12; 111, 112) passes through this reference plane (Y) is crossed with the measurement axis (6; 106) at a common intersection point (F), and wherein the at least one optical beam path (11, 12; 111, 112) generates a marker (27, 28; 127, 128) at the passage point at the reference plane (Y) in order to determine the distance (C) between the intersection point (F) and the reference plane (Y) at said reference plane (Y).

14. Method according to claim 12 or 13, *characterized in that* optical beam paths (11, 12; 111, 112) are generated successively with a time offset, whereby in particular the in a time-shifted manner generated optical beam paths (11, 12; 111, 112) are generated in a location-shifted manner as well and include a fictitious viewing angle with each other.

15. Method according to one of claims 12 to 14, *characterized in that* the determined object distance (C) is provided to operate a treatment machine in an automated manner.

Fig. 1

Fig. 2

Fig. 3

Fig. 4

Fig. 5

IV.

distance *C*

time *t*

$$C = \frac{c\,t}{2}$$

Fig. 6

V.

Fig. 7

Fig. 8

Fig. 9

Fig. 10

Europäisches Patentamt
European Patent Office
Office européen des brevets

# EUROPEAN SEARCH REPORT

Application Number

EP 23 16 3486

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2017/280989 A1 (HEEREN TAMMO [US]) 5 October 2017 (2017-10-05) <br> * abstract * <br> * claims 1-20; figures 1-5 * <br> * paragraphs [0037] - [0044], [0061] - [0077] * | 1,3-5, 11-15 <br><br> 2,6-10 | INV.<br>A61B3/13<br>A61B3/117<br>A61B3/10 |
| Y | US 2020/330276 A1 (RATHJEN CHRISTIAN [DE]) 22 October 2020 (2020-10-22) <br> * abstract * <br> * figures 1-18 * <br> * paragraph [0061] - paragraph [0103] * | 2,6-10 | |
| A | US 5 841 149 A (SPINK ROGER [CH] ET AL) 24 November 1998 (1998-11-24) <br> * the whole document * | 1-15 | |
| A | WO 2016/033590 A1 (BERESTKA JOHN [US]) 3 March 2016 (2016-03-03) <br> * abstract * | 1 | |
| A | US 5 735 283 A (SNOOK RICHARD KIETH [US]) 7 April 1998 (1998-04-07) <br> * abstract * | 1 | TECHNICAL FIELDS SEARCHED (IPC)<br><br>A61B |
| A | US 2006/203330 A1 (MOELLER GERHARD [DE] ET AL) 14 September 2006 (2006-09-14) <br> * abstract * | 1 | |

The present search report has been drawn up for all claims

| Place of search | Date of completion of the search | Examiner |
|---|---|---|
| Munich | 26 June 2023 | Tommaseo, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 3486

This annex lists the patent family members relating to the patent documents cited in the above-mentioned European search report.
The members are as contained in the European Patent Office EDP file on
The European Patent Office is in no way liable for these particulars which are merely given for the purpose of information.

26-06-2023

| Patent document cited in search report | | Publication date | Patent family member(s) | | | Publication date |
|---|---|---|---|---|---|---|
| US 2017280989 | A1 | 05-10-2017 | AU | 2017243802 | A1 | 16-08-2018 |
| | | | CA | 3013283 | A1 | 05-10-2017 |
| | | | CN | 108697319 | A | 23-10-2018 |
| | | | EP | 3435838 | A1 | 06-02-2019 |
| | | | JP | 6927991 | B2 | 01-09-2021 |
| | | | JP | 2019511273 | A | 25-04-2019 |
| | | | US | 2017280989 | A1 | 05-10-2017 |
| | | | WO | 2017168328 | A1 | 05-10-2017 |
| US 2020330276 | A1 | 22-10-2020 | EP | 3725276 | A1 | 21-10-2020 |
| | | | US | 2020330276 | A1 | 22-10-2020 |
| US 5841149 | A | 24-11-1998 | EP | 0755530 | A1 | 29-01-1997 |
| | | | EP | 0801760 | A1 | 22-10-1997 |
| | | | EP | 0822436 | A2 | 04-02-1998 |
| | | | EP | 0827002 | A2 | 04-03-1998 |
| | | | JP | H09511579 | A | 18-11-1997 |
| | | | JP | H09512922 | A | 22-12-1997 |
| | | | US | 5841149 | A | 24-11-1998 |
| | | | US | 5953114 | A | 14-09-1999 |
| | | | US | 6043890 | A | 28-03-2000 |
| | | | WO | 9527917 | A1 | 19-10-1995 |
| | | | WO | 9527918 | A2 | 19-10-1995 |
| WO 2016033590 | A1 | 03-03-2016 | EP | 3185747 | A1 | 05-07-2017 |
| | | | GB | 2544946 | A | 31-05-2017 |
| | | | JP | 2017526507 | A | 14-09-2017 |
| | | | US | 2017156591 | A1 | 08-06-2017 |
| | | | US | 2019053703 | A1 | 21-02-2019 |
| | | | US | 2020315451 | A1 | 08-10-2020 |
| | | | US | 2022400950 | A1 | 22-12-2022 |
| | | | WO | 2016033590 | A1 | 03-03-2016 |
| US 5735283 | A | 07-04-1998 | NONE | | | |
| US 2006203330 | A1 | 14-09-2006 | DE | 102005011781 | A1 | 21-09-2006 |
| | | | JP | 5094026 | B2 | 12-12-2012 |
| | | | JP | 2006247399 | A | 21-09-2006 |
| | | | US | 2006203330 | A1 | 14-09-2006 |

EPO FORM P0459

For more details about this annex : see Official Journal of the European Patent Office, No. 12/82